(19) Europäisches Patentamt
European Patent Office
Office européen des brevets

(11) **EP 0 779 259 B1**

(12) **EUROPÄISCHE PATENTSCHRIFT**

(45) Veröffentlichungstag und Bekanntmachung des Hinweises auf die Patenterteilung:
**08.12.1999 Patentblatt 1999/49**

(51) Int. Cl.$^6$: **C04B 35/65**, C04B 35/48

(21) Anmeldenummer: 96117562.7

(22) Anmeldetag: **02.11.1996**

(54) **Verfahren zur Herstellung eines oxidkeramischen Sinterkörpers und dessen Verwendung**

Process for the production of an oxide sintered body and the use thereof

Procédé de préparation de corps fritté en céramique de type oxyde et son utilisation

(84) Benannte Vertragsstaaten:
**AT BE CH DE FR GB LI**

(30) Priorität: **16.12.1995 DE 19547129**

(43) Veröffentlichungstag der Anmeldung:
**18.06.1997 Patentblatt 1997/25**

(73) Patentinhaber:
**Forschungszentrum Karlsruhe GmbH
76133 Karlsruhe (DE)**

(72) Erfinder:
• **Hausselt, Jürgen, Prof. Dr.
76726 Germersheim (DE)**
• **Hennige, Volker
76344 Eggenstein-Leopolds-hafen (DE)**
• **Ritzhaupt-Kleissl, Hans-Joachim, Dr.
69190 Walldorf (DE)**

(56) Entgegenhaltungen:
EP-A- 0 412 428          DE-A- 4 023 849
GB-A- 2 015 498

## Beschreibung

Die Erfindung betrifft ein Verfahren zur Herstellung eines oxidkeramischen Sinterkörpers gemäß dem ersten und dessen Verwendung gemäß dem siebten Patentanspruch.

Aus der japanischen Offenlegungsschrift Hei JP-A-3 198 842 ist es bekannt, maßgenaue keramische Zahnkronen herzustellen, indem ein auf Maß gearbeiteter, aus einem beim Sintern nicht schrumpfenden Material bestehender Grünkörper gebrannt wird. Mit Hilfe eines Lösungsmittels, eines Dispergiermediums und eines Bindemittels wird ein Pulver aus einer Oxidkeramik mit einem Zusatzmaterial, das durch Oxidation sein Volumen vergrößert, aufgeschlämmt. Aus der Aufschlämmung wird der Grünkörper mit der gewünschten Form erzeugt und gesintert. Als Zusatzmaterial, das durch Oxidation sein Volumen vergrößert, werden metallische Pulver wie Titan, Zirkonium, Silicium, Aluminium oder nichtoxidische Pulver wie die Carbide von Silicium, Zirkonium und Titan sowie die Nitride von Aluminium, Titan und Zirkonium vorgeschlagen. Die Oxidkeramik kann insbesondere aus Zirkoniumoxid, Aluminiumoxid, Mullit, Siliciumdioxid und Titandioxid bestehen. In den Beispielen wird als Bindemittel Methylcellulose und Acrylester-Copolymer genannt.

Die Oxidation von Metallen beim Sintern der Keramik verläuft zum Teil außerordentlich heftig, so daß die Gefahr der Zerstörung der Zahnkrone besteht. Die zur Erhöhung des Volumens vorgeschlagenen Carbide und Nitride setzen während des Sinterns unter Luft zusätzlich Gase frei, die zur Rißbildung führen können.

Ähnliche schrumpfarm sinternde Grünkörper werden in Ceramic Transactions **22** (1991) 631-646 beschrieben. Insbesondere werden die Eigenschaften von Grünkörpern aus Aluminium/Alumi-niumoxid, Aluminium/Mullit sowie Siliciumcarbid/Aluminiumoxid und die hieraus hergestellten Keramiken untersucht.

Eine weitere Beschreibung ähnlich zusammengesetzter, schrumpfarm sinternder Grünkörper findet sich in J. Am. Ceram. Soc. 76(4) 970-980 (1993).

Die DE 39 26 077 A1 betrifft keramische Sinterkörper und Verfahren zu ihrer Herstellung. Der keramische Sinterkörper ist dadurch erhältlich, daß man eine (i) keramische Matrix, (ii) eine Mischung aus einem siliciumorganischen Polymer und (iii) ein Metall oder eine intermetallische Verbindung der vierten bis sechsten Nebengruppe mischt und unter Inertgas oder reduzierender Gasatmosphäre sintert. Gemäß diesem Verfahren bilden sich in der keramischen Matrix Einlagerungen von Hartstoffteilchen; diese Hartstoffteilchen entstehen beim Sintern unter inerten oder reduzierenden Bedingungen infolge einer Reaktion des Metalls oder der intermetallischen Verbindung mit den Zersetzungsprodukten des siliciumorganischen Polymers, insbesondere mit dem im siliciumorganischen Polymer enthaltenen Kohlenstoff.

Das Verfahren ist nur für nichtoxidische Keramiken anwendbar, obwohl an einer Stelle auch eine Oxidkeramik ($SiO_2$) erwähnt wird. Bei diesem Verfahren muß verhindert werden, daß die Zersetzungsprodukte des siliciumorganischen Polymers oxidiert werden, weil sonst die gewünschten eingelagerten Hartstoffteilchen nicht entstehen. Aus demselben Grund ist eine Atmosphäre aus inertem oder reduzierenden Gas während des Sinterns unabdingbar.

Das in der DE 39 26 077 A1 beschriebene Verfahren ist Gegenstand weiterer Veröffentlichungen (Journal of Materials Science **27** (1992) 1053-1060 und Journal of the European Ceramic Society **11** (1993) 105-113).

Über den Einsatz siliciumorganischer Polymere zur Herstellung von Keramiken berichtet eine Veröffentlichung in Mat. Res. Soc. Symp. Proc. Vol. 249, pp 3-13 (1992 Materials Research Society). Bei den Keramiken handelt es sich nahezu ausschließlich um Nichtoxid-Keramiken, bei denen die Sinterung unter inerten oder reduzierenden Bedingungen durchgeführt wird.

Aufgabe der Erfindung ist, ein weiteres Verfahren zur Herstellung eines oxidkeramischen Sinterkörpers anzugeben, bei dem der Schrumpf nach der Sinterung minimiert oder definiert eingestellt werden kann, so daß sich der Sinterkörper als Zahnfüllung verwenden läßt. Bei dem Verfahren soll auf den Einsatz der heftig reagierenden Metalle verzichtet werden.

Die Aufgabe wird erfindungsgemäß durch das im ersten Patentanspruch beschriebene Verfahren gelöst. Die weiteren Patentanspüche geben bevorzugte Ausgestaltungen des erfindungsgemäßen Verfahrens an. Die Verwendung des nach dem erfindungsgemäßen Verfahren hergestellten oxidkeramischen Sinterkörpers ist Gegenstand von Anspruch 7.

Erfindungsgemäß wird aus einer pulverförmigen Oxidkeramik, einer pulverförmigen intermetallischen Verbindung und einem reinen oder in einem Lösungsmittel gelösten siliciumorganischen Polymer eine homogene Mischung hergestellt.

Als Oxidkeramiken kommen die typischen Strukturkeramiken wie z. B. Zirconiumoxid oder Aluminiumoxid in Betracht. Als intermetallische Verbindungen werden bevorzugt Silicide oder Aluminide wie z. B. Zirconiumsilicid ($ZrSi_2$), Aluminiumsilicid ($Al_4Si_3$), Titansilicid ($TiSi_2$) oder Titanaluminid ($TiAl_x$) eingesetzt. Die intermetallischen Verbindungen haben gegenüber den Metallen den Vorzug, daß die Oxidation wesentlich weniger heftig verläuft. Silicide und Aluminide gehören zu den intermetallischen Verbindungen, die beim Sintern unter oxidierender Atmosphäre zu Bestandteilen des oxidkeramischen Sinterkörpers oxidiert werden; sie sind aus diesem Grund vorteilhaft.

Erfindungsgemäß dient das siliciumorganische Polymer zugleich als Bindemittel und als Bestandteil, der beim Sintern unter oxidativen Bedingungen eine hohe oxidkeramische Ausbeute ergibt; es trägt somit allenfalls unwesentlich zum Schrumpf beim Sintern bei, so daß sein Anteil nicht mit einem übermäßig hohen Anteil der intermetallischen Verbindung kompensiert werden muß. Besonders bevorzugt werden Polysiloxane als siliciumorganisches Polymer, weil deren Oxidation zu Siliciumdioxid führt.

Das siliciumorganische Polymer wird in der Regel vor der Herstellung der Mischung mit einem geeigneten Lösungsmittel gelöst. Geeignete Lösungsmittel sind beispielsweise Ethanol oder Aceton.

Durch geeignete Wahl der drei Komponenten Oxidkeramik, intermetallische Verbindung und siliciumorganisches Polymer sowie deren Konzentration in der Mischung läßt sich ein Grünkörper mit minimiertem Schrumpf herstellen.

Die Menge an intermetallischer Verbindung, die notwendig ist, um den Sinterschrumpf auszugleichen, ergibt sich aus der folgenden Abschätzung für den linearen Sinterschrumpf S:

$$S = \sqrt[3]{[(1 + \Sigma V_i \Delta V_i) \cdot \delta_{Grün}/\delta_{Sinter}]} - 1$$

mit

$V_i$ Volumanteil der Komponente i in der homogenen Mischung
$\Delta V_i$ relative Volumenänderung der Komponente i
$\delta_{Grün}$ Dichte des Grünkörpers
$\delta_{Sinter}$ Dichte des Sinterkörpers.

Für einen Sinterschrumpf S = 0 kann diese Gleichung bei Verwendung von Polymethylsilsesquioxan (PMSS) als siliciumorganisches Polymer aufgelöst werden nach dem benötigten Anteil ($V_{intermet.V.}$) an intermetallischer Verbindung:

$$V_{intermet.V.} = \frac{\delta_{Sinter}/\delta_{Grün} - V_{PMSS} \cdot \Delta V_{PMSS} - 1}{\Delta V_{intermet.V.}}$$

Ist das siliciumorganische Polymer in einem Lösungsmittel gelöst, kann die Formgebung des Grünkörpers nach zwei verschiedenen Methoden erfolgen. Es besteht die Möglichkeit, die homogene Mischung der drei Komponenten in eine Form einzufüllen und das Lösungsmittel etwa durch Verdampfen zu entfernen. Alternativ kann aus der homogenen Mischung das Lösungsmittel durch Aodestillieren entfernt werden, so daß ein getrocknetes Granulat entsteht. Das getrocknete Granulat kann einem geeigneten Formgebungsverfahren, etwa Kalt- oder Warmpressen, Spritzgießen oder Extrudieren unterworfen werden.

Soll die homogene Mischung kein Lösungsmittel enthalten, so besteht die Möglichkeit, die intermetallische Verbindung und die Oxidkeramik in flüssigem siliciumorganischen Polymer zu suspendieren und die Suspension in eine Form einzufüllen, in der das siliciumorganische Polymer quervernetzt wird. Alternativ kann ein festes siliciumorganisches Polymer aufgeschmolzen werden; bei Schmelztemperatur wird dann wiederum eine Suspension der intermetallischen Verbindung und der Oxidkeramik hergestellt. Die Suspension kann in eine Form gefüllt und abgekühlt werden.

Die Formgebung wird bei allen genannten Varianten des erfindungsgemäßen Verfahrens dadurch vereinfacht, daß der Anteil an siliciumorganischem Polymer zumindest zwischen etwa 5 und 50 Vol-% variiert werden kann.

Der geformte Grünkörper wird anschließend gesintert. Das Sintern erfolgt unter oxidierender Atmosphäre, in der Regel also unter Luftatmosphäre. Die Sintertemperaturen sollen im Bereich zwischen 1450° C und 1650° C liegen. Die Sinterdauer liegt im allgemeinen zwischen 10 und 24 Stunden.

Die Erfindung wird im folgenden anhand eines Ausführungsbeispiels näher erläutert.

43,5 g Zirconiumdisilicid und 46,8 g Zirconiumdioxid wurden 24 h lang in einer Planetenkugelmühle mischgemahlen. Anschließend wurden 9,75 g Polymethylsilsesquioxan (PMSS) in Ethanol gelöst zugegeben. Danach wurde das Lösungsmittel im Vakuum abdestilliert.

Aus dem getrockneten Pulver wurden dann durch Pressen zylindrische Grünkörper mit einer Dichte von etwa 81 % der theoretischen Dichte TD hergestellt. Diese Grünkörper wurden zunächst in einem ersten Schritt unter Luft einer Temperatur von etwa 600° C, dann in einem zweiten Schritt maximal 1200° C ausgesetzt. Im ersten Schritt setzte sich das PMSS zu Siliciumdioxid um; im zweiten Schritt wurde Zirconiumdisilicid zu Zirconiumdioxid und Siliciumdioxid aufoxidiert.

Im Anschluß an diese Temperaturbehandlung wurden die Grünkörper, deren Volumen aufgrund der Oxidation des Zirconiumdisilicids stark zugenommen hat, bei 1600° C unter Luft zu dichten Sinterkörpern gesintert. Eine Röntgenanalyse ergab, daß die Sinterkörper in der Hauptsache aus Zirconiumsilicat ($ZrSiO_4$) bestanden. Die Dichte betrug ca. 93 % der theoretischen Dichte. Der lineare Schrumpf des Sinterkörpers bezogen auf den Grünkörper ist kleiner als 1 %. Der Sinterkörper besitzt eine Härte (nach Vickers) von etwa 1020 HV10.

**Patentansprüche**

1. Verfahren zur Herstellung eines oxidkeramischen Sinterkörpers, bei dem

   a) eine homogene Mischung aus einer pulverförmigen Oxidkeramik, einer pulverförmigen intermetallischen Verbindung und einem siliciumorganischen Polymer hergestellt,
   b) aus der homogenen Mischung ein Grünkörper geformt und
   c) der Grünkörper unter oxidierender Atmosphäre zu dem keramischen Sinterkörper gesintert wird.

2. Verfahren nach Anspruch 1, bei dem das siliciumorganische Polymer in einem Lösungsmittel gelöst ist.

3. Verfahren nach Anspruch 2, bei dem der Grünkörper geformt wird, indem die homogene Mischung in eine Form eingefüllt und das Lösungsmittel entfernt wird.

4. Verfahren nach Anspruch 2, bei dem der Grünkörper geformt wird, indem aus der homogenen Mischung das Lösungsmittel durch Abdestillieren entfernt wird, so daß ein getrocknetes Granulat entsteht, und das Granulat einem Formgebungsverfahren unterworfen wird.

5. Verfahren nach einem der Ansprüche 1 bis 4, bei dem als intermetallische Verbindung ein Silicid oder ein Aluminid eingesetzt wird.

6. Verfahren nach einem der Ansprüche 1 bis 5, bei dem die Anteile der Oxidkeramik, der intermetallischen Verbindung und des siliciumorganischen Polymers in der homogenen Mischung durch nach der folgenden Beziehung bestimmt werden:

$$S = [(1 + \Sigma V_i \Delta V_i) \cdot \delta_{Grün}/\delta_{Sinter}]^{(1/3)} - 1$$

wobei S den linearen Sinterschrumpf, $V_i$ den Volumanteil der Komponente i in der homogenen Mischung, $\Delta V_i$ die relative Volumenänderung der Komponente i, $\delta_{Grün}$ die Dichte des Grünkörpers und $\delta_{Sinter}$ Dichte des Sinterkörpers bedeuten.

7. Verwendung des nach einem der Ansprüche 1 bis 6 hergestellten oxidkeramischen Sinterkörpers als Zahnfüllmaterial.

**Claims**

1. Method of producing an oxide-ceramic sintered body, wherein

   a) a homogeneous mixture is produced from a pulverulent oxide ceramic, a pulverulent intermetallic compound and a silicon-organic polymer,
   b) a green body is shaped from the homogeneous mixture, and
   c) the green body is sintered in an oxidising atmosphere to form the ceramic sintered body.

2. Method according to claim 1, wherein the silicon-organic polymer is dissolved in a solvent.

3. Method according to claim 2, wherein the green body is shaped as a result of the homogeneous mixture being introduced into a mould, and the solvent being removed.

4. Method according to claim 2, wherein the green body is shaped as a result of the solvent being removed from the homogeneous mixture by distillation so that a dried granulate is produced, and the granulate is subjected to a form-shaping process.

5. Method according to one of claims 1 to 4, wherein a silicide or an aluminide is used as the intermetallic compound.

6. Method according to one of claims 1 to 5, wherein the proportions of the oxide ceramic, the intermetallic compound and the silicon-organic polymer in the homogeneous mixture are determined by the following equation:

$$S = [(1 + \Sigma V_i \Delta V_i) \cdot \delta_{green}/\delta_{sinter}]^{(1/3)} - 1,$$

in which S signifies the linear sintering shrinkage, $V_i$ signifies the proportion by volume of the component i in the homogeneous mixture, $V_i$ signifies the relative change in volume of the component i, $\delta_{green}$ signifies the density of the green body, and $\delta_{sinter}$ signifies the density of the sintered body.

7. Use of the oxide-ceramic sintered body, which is produced according to one of claims 1 to 6, as a tooth filling material.

**Revendications**

1. Procédé de fabrication d'un corps fritté oxydocéramique dans lequel :

   • on produit un mélange homogène constitué d'une oxydocéramique pulvérulente, d'un composé intermétallique pulvérulent et d'un polymère d'organosilicium,
   • on façonne une ébauche à partir du mélange homogène, et
   • on fritte l'ébauche dans une atmosphère oxydante pour obtenir le corps fritté céramique.

2. Procédé selon la revendication 1,
   dans lequel
   le polymère d'organosilicium est dissous dans un solvant.

3. Procédé selon la revendication 2,
   dans lequel
   on façonne l'ébauche en versant le mélange homogène dans un moule et en retirant le solvant.

4. Procédé selon la revendication 2,
   dans lequel
   on façonne l'ébauche en retirant par distillation le solvant du mélange homogène si bien qu'il apparaît un granulé séché, et on soumet le granulé à un procédé de façonnage.

5. Procédé selon l'une des revendications 1 à 4,
   dans lequel
   on utilise comme composé intermétallique un siliciure ou un aluminure.

6. Procédé selon l'une des revendications 1 à 5,
   dans lequel
   on détermine les proportions de l'oxydocéramique, du composé intermétallique et du polymère d'organosilicium dans le mélange homogène par la relation suivante :

$$S = [(1 + \Sigma V_i \Delta V_i) \cdot \delta_{ébauche}/\delta_{corps\ fritté}]^{(1/3)} - 1$$

dans laquelle S représente la rétraction linéaire au frittage, $V_i$ la proportion volumique du composant i dans le mélange homogène, $\Delta Vi$ la modification relative du volume du composant i, $\delta_{ébauche}$ la densité de l'ébauche et $\delta_{densité}$ densité du corps fritté.

7. Utilisation du corps fritté oxydocéramique produit selon l'une des revendications 1 à 6 comme matériau d'obturation dentaire.